# NEUE EUROPÄISCHE PATENTSCHRIFT

(11) **EP 0 403 618 B2**
(45) Veröffentlichungstag und Bekanntmachung des Hinweises auf die Entscheidung über den Einspruch: **28.04.2004**
(45) Hinweis auf die Patenterteilung: 01.02.1995
(21) Anmeldenummer: 89913190.8
(22) Anmeldetag: 12.12.1989
(51) Int. Cl.: C07C 251/60, C07C 255/64, A01N 35/10, A01N 43/00, C07D 307/14, C07D 307/81, C07D 215/12, C07D 261/10, C07D 213/52, C07D 333/22

(54) **ALDIMINO- ODER KETIMINO-OXY-ORTHO-TOLYLACRYLSÄURE-METHYLESTER, IHRE HERSTELLUNG UND DIESE ENTHALTENDE FUNGIZIDE**
METHYL ESTERS OF ALDIMINO- OR KETIMINO-OXY-ORTHO-TOLYLACRYLIC ACID, MANUFACTURING PROCESS AND FUNGICIDES CONTAINING THEM
ESTERS METHYLIQUES ALDIMINO- OU CETIMINO-OXY-ORTHO-TOLYLACRYLIQUES, MODE DE FABRICATION ET FONGICIDES CONTENANT A CES COMPOSES

(30) Priorität: 29.12.1988 CH 484988; 03.11.1989 CH 397589
(43) Veröffentlichungstag der Anmeldung: 27.12.1990
(73) Patentinhaber: Bayer Aktiengesellschaft, 51368 Leverkusen (DE)
(72) Erfinder: ISENRING, Hans, Peter, CH-4450 Sissach (CH); TRAH, Stephan, D-7800 Freiburg (DE); WEISS, Bettina, CH-4052 Basel (CH)
(86) Internationale Anmeldenummer: PCT/CH1989/000216
(87) Internationale Veröffentlichungsnummer: WO 1990/007493

(56) Entgegenhaltungen:
- EP-A- 370 629
- GB-A- 2 025 407

## Beschreibung

Die vorliegende Erfindung betrifft substituierte 2-Phenyl-3-methoxyacrylsäuremethylester der allgemeinen Formel 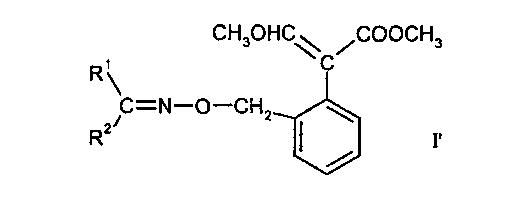 in welcher R¹ und R² die folgenden Bedeutungen haben:

| **R**^{**1**} | **R**^{**2**} |
|---|---|
| 3,5-Di(trifluormethyl)-phenyl | Methyl |
| 2-Fluor-5-methylphenyl | Methyl |
| 2-Benzofuranyl | Methyl |
| 2-Chinolinyl | Wasserstoff |
| 4-Fluorphenyl | Methyl |
| 4-Nitrophenyl | Methyl |
| 4-Chlorphenyl | Methyl |
| 4-Chlorphenyl | Aethyl |
| β-Phenyläthenyl | Methyl |
| o-Tolyl | Methyl |
| p-Anisyl | Methyl |
| 4-Biphenylyl | Methyl |
| Cyclopropyl | Cyclopropyl |
| β-(2-Furyl)-äthenyl | Methyl |
| β-(3,4,5-Trimethoxy-phenyl)-äthenyl | Methyl |
| α, α, α,-Trifluor-m-tolyl | Methyl |
| 3-Bromphenyl | Methyl |
| 2-Chlorphenyl | Methyl |
| 3-Nitrophenyl | Methyl |
| 5-Chlor-3-methyl-2-benzothienyl | Methyl |
| 3-Chlorphenyl | Methyl |
| 3,4-Dichlorphenyl | Methyl |
| 2,4-Dichlorphenyl | Methyl |
| 2,4-Dichlorphenyl | 3-Pyridylmethyl |
| 3-Methyl-2-benzothienyl | Methyl |
| 2,5-Dichlorphenyl | Methyl |
| 4-Aethylphenyl | Methyl |
| 3-Aethylphenyl | Methyl |
| 3,4-Dimethylphenyl | Methyl |
| 6-Methyl-2-naphthyl | Methyl |
| 4-Difluormethoxyphenyl | Methyl |
| 4-Chlorphenyl | Neopentyl |
| 3,5-Di(trifluormethyl)-phenyl | Aethyl |
| 3-Phenanthryl | Methyl |
| 2-Fluorenyl | Methyl |
| Isopropyl | Methyl |
| 4-Chlorphenyl | n-Propyl |
| 4-Chlorphenyl | Cyclopropyl |
| 3-Chlorphenyl | Aethyl |
| 4-Fluorphenyl | Aethyl |
| 4-Bromphenyl | Aethyl |
| 4-tert.-Butylphenyl | Methyl |
| Cyclopropyl | Methyl |
| 2-Chinolinylmethyl | Methyl |
| 3-Fluor-5-trifluormethyl-phenyl | Methyl |
| 3,5-Difluorphenyl | Methyl |
| 3,5-Difluorphenyl | Aethyl |
| 2-Fluorphenyl | Aethyl |
| 3,4-Dimethoxyphenyl | Methyl |
| p-Tolyl | Aethyl |
| 3-Trifluormethoxyphenyl | Methyl |
| 3-Fluor-5-trifluormethyl-phenyl | Aethyl |
| Cyclohexyl | Methyl |
| 4-Chlorphenyl | Trifluormethyl |
| 3,4-Dimethoxybenzyl | Methyl |
| α, α, α,-Trifluor-m-tolyl | Trifluormethyl |
| α, α, α,-Trifluor-m-tolyl | Aethyl |
| 3,5-Dichlorphenyl | Methyl |
| β-(2-Naphthyl)-äthenyl | Trifluormethyl |
| Methyl | Methyl |
| α, α, α,-Trifluor-m-tolyl | n-Propyl |
| α, α, α,-Trifluor-m-tolyl | Cyclopropyl |
| 2-Chlor-5-trifluormethyl-phenyl | Methyl |
| 2-Methylthio-5-trifluormethylphenyl | Methyl |
| 4-Chlor-3-trifluormethyl-benzyl | Methyl |
| 4-Methoxybenzyl | Methyl |
| α, α, α,-Trifluor-m-tolyl | Isopropyl |
| α, α, α,-Trifluor-m-tolyl | α, α, α,-Trifluor-m-tolyl |
| 4-Äthoxyphenyl | Methyl |
| 3-Chlor-4-fluorphenyl | Methyl |
| 2,3-Dichlorphenyl | Methyl |
| p-Tolyl | Trifluormethyl |
| 4-(2,4-Dichlorphenoxy)-phenyl | Methyl |
| 4-(4-Nitrophenoxy)phenyl | Methyl |
| 4-(4-Methoxyphenoxy)-phenyl | Methyl |
| 3,4-Methylendioxyphenyl | Methyl |
| 7-Benzodioxanyl | Methyl |
| 3-Bromphenyl | Aethyl |
| 3-Fluor-5-trifluormethylphenyl | Cyclopropyl |
| 2,3,4-Trichlorphenyl | Methyl |
| 4-Chlor-3-methylphenyl | Methyl |
| p-Tolyl | n-Propyl |
| 4-Phenoxyphenyl | Cyclopropyl |
| 3-Bromphenyl | Cyclopropyl |
| 3-Chlorphenyl | Cyclopropyl |
| 3-Fluorphenyl | Cyclopropyl |
| 3-Bromphenyl | n-Propyl |
| 3-Chlorphenyl | n-Propyl |
| 4-Fluor-3-trifluormethylphenyl | n-Propyl |
| 3-Fluorphenyl | n-Propyl |
| 4-Phenoxyphenyl | n-Propyl |
| 3-Fluor-5-trifluormethylphenyl | n-Propyl |
| 3-Bromphenyl | Trifluormethyl |
| 3-Chlorphenyl | Trifluormethyl |
| 4-Phenoxyphenyl | Trifluormethyl |
| 3-Bromphenyl | Isopropyl |
| 3-Chlorphenyl | Isopropyl |
| 4-Phenoxyphenyl | Isopropyl |
| 4-Fluor-3-trifluormethylphenyl | Cyclopropyl |
| 4-Fluorphenyl | Cyclopropyl |
| 4-(n-Propyl)-phenyl | Methyl |
| 4-Methoxy-3-nitrophenyl | Methyl |
| 2,4-Dimethoxyphenyl | Methyl |
| 4-Fluor-3-trifluormethylphenyl | Isopropyl |
| 3-Iodphenyl | Methyl |
| 4-Iodphenyl | Methyl |
| 2-(4-Methoxyphenyl)äthyl | Methyl |
| 1,4,8-Trimethyl-nona-1,3,7-trienyl | Methyl |
| 1-Methyl-2-(3,4-methylendioxyphenyl)äthyl | Methyl |

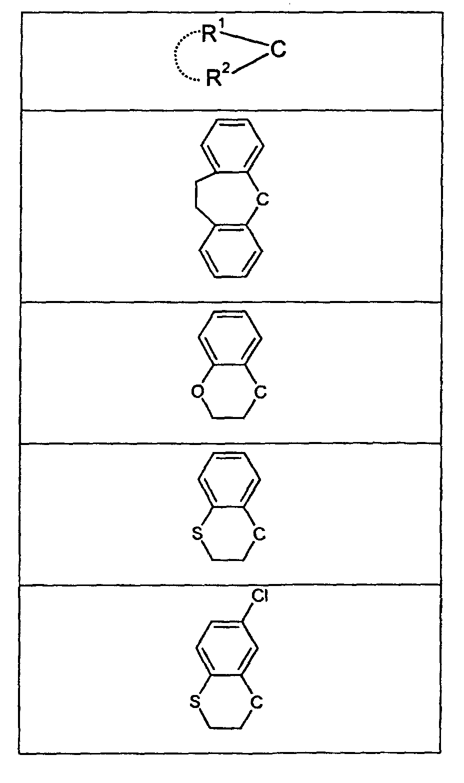

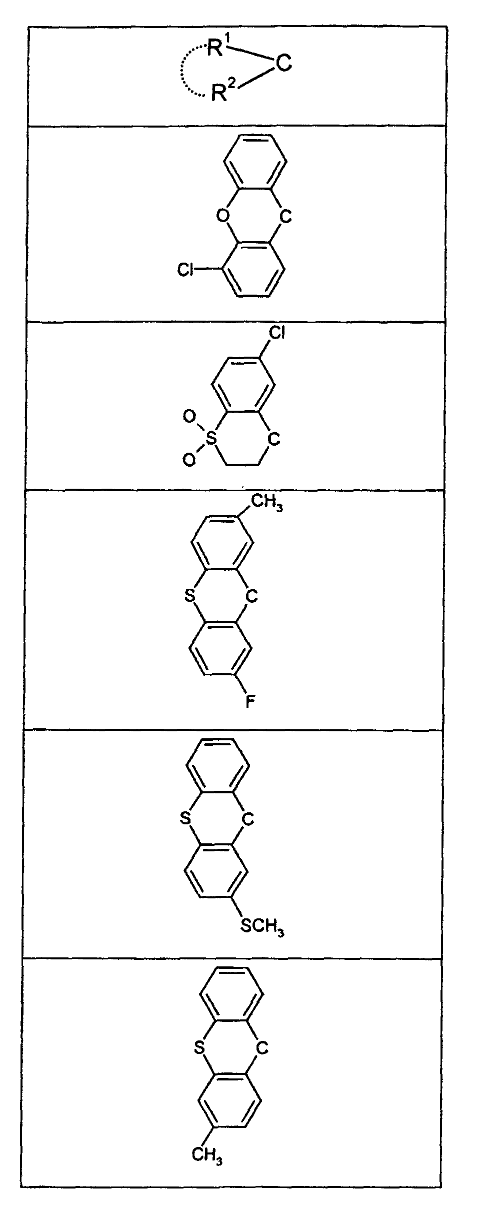

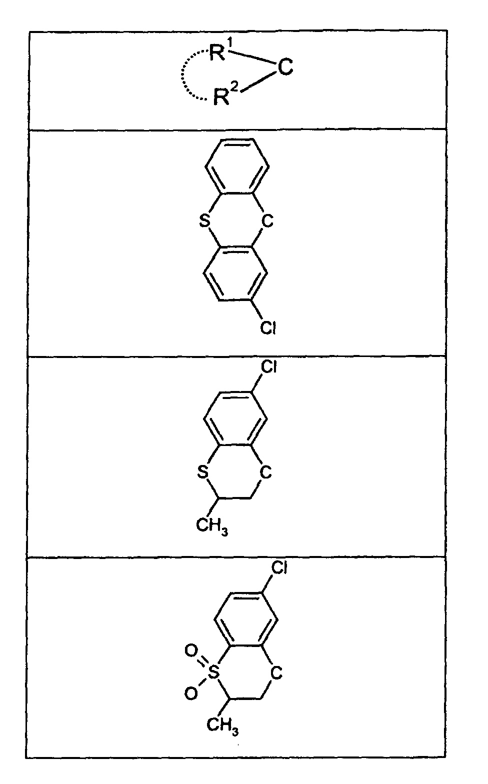

Die erfindungsgemäßen Verbindungen besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der erfindungsgemäßen Verbindungen, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

Falls in den Verbindungen der Formel I' asymmetrische Kohlenstoffatome vorliegen, treten die Verbindungen in optisch aktiver Form auf. Allein aufgrund des Vorhandenseins der aliphatischen und der Imino-Doppelbindung treten die Verbindungen auf jeden Fall in der [E]- oder [Z]-Form auf. Ferner kann Atropisomerie auftreten. Die Formel I' soll all diese möglichen isomeren Formen sowie deren Gemische, z.B. racemische Gemische und beliebige [E/Z]-Gemische, umfassen.

In der unter Art. 54(3) EPUe zitierten EP-A 370 629, die der Schutzumfang vorliegender Erfindung abgegrenzt wurde, werden Fungizide/Insektizide auf Basis von Methoxyacrylat-Derivaten mit teilweise ähnlicher Struktur beschrieben.

Im Allgemeinen sind die [E]-Formen der Verbindungen der Formel I' gegenüber den [Z]-Formen bevorzugt.

Das erfindungsgemäße Verfahren zur Herstellung der erfindungsgemäßen Verbindungen ist dadurch gekennzeichnet, dass man ein Oxim X-OH der allgemeinen Formel 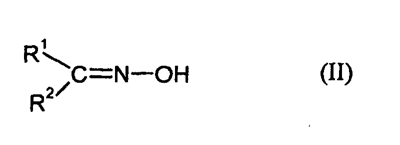 worin
- R¹ und R²: die oben angegebenen Bedeutungen besitzen,
mit einem Benzylalkoholderivat der allgemeinen Formel 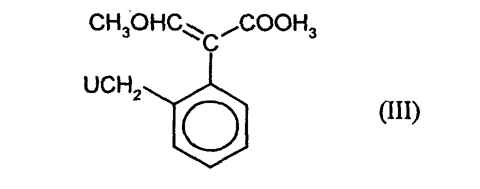 worin
- U: eine Abgangsgruppe bedeutet,
umsetzt.

Bei dieser Umsetzung handelt es sich um eine nucleophile Substitution, die unter den diesbezüglich üblichen Reaktionsbedingungen durchgeführt werden kann. Unter der im Benzylalkoholderivat der Formel (III) vorhandenen Abgangsgruppe U ist vorzugsweise Chlor, Brom, Iod, Mesyloxy oder Tosyloxy zu verstehen. Die Umsetzung erfolgt zweckmäßigerweise in einem inerten organischen Verdünnungsmittel, wie einem cyclischen Ether, z.B. Tetrahydrofuran oder Dioxan, Aceton, Dimethylformamid oder Dimethylsulfoxid, in Gegenwart einer Base, wie Natriumhydrid, Natrium- oder Kaliumcarbonat, eines tertiären Amins, z.B. eines Trialkylamins, insbesondere Diazabicyclononan oder Diazabicycloundecan, oder Silberoxid, bei Temperaturen zwischen -20°C und 80°C, vorzugsweise im Temperaturbereich von 0°C bis 20°C.

Als Alternative kann die Umsetzung unter Phasentransferkatalyse in einem organischen Lösungsmittel, wie beispielsweise Methylenchlorid, in Gegenwart einer wässrigen basischen Lösung, z.B. Natriumhydroxidlösung, sowie eines Phasentransferkatalysators, wie beispielsweise Tetrabutylammoniumhydrogensulfat, bei Raumtemperatur erfolgen.

Die Isolierung und Reinigung der so hergestellten Verbindungen der Formel I' kann nach an sich bekannten Methoden erfolgen. Ebenfalls nach an sich bekannten Methoden können allfällig erhaltene Isomerengemische, z.B. E/Z-Isomerengemische, in die reinen Isomeren aufgetrennt werden, beispielsweise durch Chromatographie oder fraktionierte Kristallisation.

Die als Ausgangsmaterialien im erfindungsgemäßen Verfahren verwendeten Oxime X-OH, z.B. der Formel (II), sind entweder bekannt oder können nach an sich bekannten Methoden hergestellt werden, beispielsweise durch Umsetzung der entsprechenden Carbonylverbindung R¹R²C=O mit Hydroxylaminhydrochlorid in Gegenwart einer Base, z.B. Natrium- oder Kaliumhydroxid oder Pyridin. Weitere Methoden finden sich in Houben-Weyl, "Methoden der Organischen Chemie", Band X/4, Seiten 3 - 308 (1968) ("Herstellung und Umwandlung von Oximen").

Ebenfalls können die Ausgangsmaterialien der Formel (III) auf an sich bekannte Weise hergestellt werden, z.B. wie in der europäischen Patentpublikation Nr. 203.606 (BASF) und in der dort zitierten Literatur beschrieben.

Die erfindungsgemäßen Verbindungen besitzen fungizide Wirkung und können dementsprechend zur Bekämpfung von Pilzen in der Landwirtschaft, im Gartenbau sowie in der Holzverarbeitung Verwendung finden. Sie eignen sich insbesondere zur Hemmung des Wachstums oder zu Vernichtung von phytopathogenen Pilzen auf Pflanzenteilen, z.B. Blättern, Stängeln, Wurzeln, Knollen, Früchten oder Blüten, und auf Saatgut sowie von im Erdboden auftretenden Schadpilzen. Ferner können mit den erfindungsgemäßen Verbindungen holzabbauende und holzverfärbende Pilze bekämpft werden. Die erfindungsgemäßen Verbindungen sind beispielsweise wirksam bei der Bekämpfung von Pilzen der Klassen Deuteromycetes, Ascomycetes, Basidiomycetes and Phycomycetes.

Besonders eignen sich die erfindungsgemäßen Verbindungen zur Bekämpfung der folgenden Schaderreger:
Echte Mehltaupilze (z.B. Erysiphe graminis, Erysiphe cichoracearum, Podosphaera leucotricha, Unicinula necator, Sphaerotheca spp.)
Rostpilze (z.B. Puccinia tritici, Puccinia recondita, Puccinia hordei, Puccinia coronata, Puccinia striiformos, Puccinia arachidis, Hermileia vastatrix, Urommyces fabae)
Schorfpilze (z.B. Venturia inaequalis)
Cercospora spp. (z.B. Cercospora arachidicola, Cercospora beticola)
Mycosphaerella spp. (z.B. Mycosphaerella fijiensis)
Atemaria spp. (z.B. Alternaria brassicae, Alternaria mali)
Septoria spp. (z.B. Septoria nodorum)
Heminthosporium spp. (z.B. Helminthosporium teres, Helminthosporium oryzae)
Plasmopara spp. (z.B. Plasmopara viticola)
Pseudoperonospora (z.B. Pseudoperonospora cubensis)
Phythophtora spp. (z.B. Phytophtora infestans)
Pseudocercosporella spp. (z.B. Pseudocercosporella herpotrichoides)
Piricularia spp. (z.B. Piricularia oryzae)

Ferner wirken die Verbindungen beispielsweise gegen Pilze der Gattungen Tilletia, Ustilago, Rhizotonia, Verticillium, Fusarium, Pythium, Gaeumannomyces, Sclerotinia, Monilia, Botrytis, Peronospora, Bremia, Gloeosporium, Cercosporidium, Penicillium, Ceratocystis, Rhynchosporium, Pyrenophora, Diaporthe, Ramularia und Leptosphaeria. Gewisse Vertreter der erfindungsgemäßen Verbindungen besitzen zudem Wirkung gegen holzschädigende Pilze, wie beispielsweise der Gattungen, Coniophora, Gloeophyllum, Poria, Merulius, Trametes, Aureobasidium, Sclerophoma und Trichoderma.

Die erfindungsgemäßen Verbindungen zeichnen sich durch prophylaktische und kurative Wirkung aus. Die erfindungsgemäßen Verbindungen wirken gegen phytopathogene Pilze unter Gewächshausbedingungen bereits bei Konzentrationen von 0,5 mg bis 500 mg Wirkstoff pro Liter Spritzbrühe. Im Freiland werden vorteilhaft Dosierungen von 20 g bis 1 kg Wirkstoff der Formel I' pro Hektar und Behandlung zur Anwendung gebracht. Zur Bekämpfung von samen- oder bodenbürtigen Pilzen im Beizverfahren werden mit Vorteil Dosierungen von 0,01 g bis 1,0 g Wirkstoff der Formel I' pro kg Samen verwendet.

Die erfindungsgemäßen Verbindungen können zu verschiedenen Mitteln, z.B. Lösungen, Suspensionen, Emulsionen, emulgierbaren Konzentraten und pulverförmigen Präparaten, formuliert werden. Die erfindungsgemäßen fungiziden Mittel sind dadurch gekennzeichnet, dass sie eine wirksame Menge mindestens einer Verbindung der allgemeinen Formel I', wie oben definiert, sowie Formulierungshilfsstoffe enthalten. Die Mittel enthalten zweckmäßigerweise zumindest einen der folgenden Formulierungshilfsstoffe:
Feste Trägerstoffe; Lösungs- bzw. Dispersionsmittel; Tenside (Netzmittel und Emulgatoren); Dispergiermittel (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate: organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen in wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chlorethylene und Methylenchlorid; aliphatische Kohlenwasserstoffe, wie Cyclohexan und Paraffine, z.B. Erdölfraktionen; Alkohole, wie Butanol und Glykol, sowie deren Ether und Ester; Ketone, wie Aceton, Methylethylketon, Methylisobutylketon und Cyclohexanon; und stark polare Lösungs- bzw. Dispersionsmittel, wie Dimethylformamid, N-Methylpyrrolidon und Dimetyhylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netzmittel und Emulgatoren) können nicht ionische Verbindungen sein, wie Kondensationsprodukte von Fettsäuren, Fettalkoholen oder fettsubstituierten Phenolen mit Ethylenoxid, Fettsäureester und -ether von Zuckern oder mehrwertigen Alkoholen; die Produkte, die aus Zuckern oder mehrwertigen Alkoholen durch Kondensation mit Ethylenoxid enthalten werden; Blockpolymere von Ethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat; und Butylnaphthalinsulfonate; und komplexere Fettsulfonate, z.B. die Amidkondensationsprodukte von Ölsäure und N-Methyltaurin und das Natriumsulfonat von Dioctylsuccinat.

Die Tenside können schließlich kationische Verbindungen sein, wie Alkyldimethylbenzylammoniumchloride, Dialkyldimethylammoniumchloride, Alkyltrimethylammoniumchloride und ethoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd und Sulfitablaugen.

Als Dispergiermittel, die sich insbesondere als Verdickungs- bzw. Antiabsetzmittel eignen, können z.B. Methylcellulose, Carboxymethylcellulose, Hydroxyethylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidether und Soyaepoxide, Antioxadantien, z.B. Gallusäureester und Butylhydroxytoluol; UV-Absorber, z.B. substituierte Benzophenone, Diphenylacrylnitrilsäureester und Zimtsäureester; und Deskativatoren, z.B. Salze der Ethylendiamintetraessigsäure und Polyglykole.

Die erfindungsgemäßen fungiziden Mittel können neben den Wirkstoffen der Formel I' auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreitung des Wirkungsspektrum oder zur spezifischen Beeinflussung des Pflanzenwachstums.

Im Allgemeinen enthalten die erfindungsgemäßen fungiziden Mittel, je nach deren Art, zwischen 0,0001 und 85 Gewichtsprozent an erfindungsgemäßer Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigen Bereich des obigen Konzentrationsintervalls. Emulgierbare Konzentrate enthalten im Allgemeinen 5 bis 85 Gewichtsprozent vorzugsweise 25 bis 75 Gewichtsprozent, der erfindungsgemäßen Verbindung(en). Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemäßen fungiziden Mittel können dadurch hergestellt werden, dass man mindestens eine erfindungsgemäße Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder Suspendieren in geeigneten Lösungs- bzw. Dispersionsmittel, eventuell unter Verwendung von Tensiden als Netzmittel oder Emulgatoren oder von Dispergiermitteln, durch Verdünnen bereits vorbereiteter emulgierbarer Konzentrate mit Lösungs- bzw. Dispergiermitteln usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, überführt werden können.

Die erfindungsgemäßen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemäße Verbindung in einem mit Wasser nicht mischbaren Lösungsmitteln, wie beispielsweise einem alicyclischen Keton, gelöst werden, das zweckmäßigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise enthält man emulgierbare Konzentrate bzw. gebrauchsfertige Emulsionen.

Die Verwendung der erfindungsgemäßen Mittel kann nach den im Pflanzenschutz bzw. in der Landwirtschaft üblichen Applikationsmethoden erfolgen. Das erfindungsgemäße Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemäßen Verbindung bzw. eines erfindungsgemäßen Mittels behandelt.

Die nachstehenden Beispiele illustrieren die Erfindung.

### I. Herstellung der Wirkstoffe der Formel I'

### Beispiel 1 (Herstellungsbeispiel außerhalb des stofflichen Umfangs der Erfindung)

Zu 0,19 g (7,7 mMol) Natriumhydrid in 20 ml Dimethylformamid gibt man bei 0°C 2,0 g (7,0 mMol) 2-(α-Brom-o-tolyl)-3-methoxyacrylsäure-methylester und 0,95 g (7,0 mMol) Acetophenonoxim. Nach 10-minütigem Rühren des Reaktionsgemisches wird gesättigte Natriumhydrogencarbonatlösung zugegeben und das Gemisch dreimal mit Ethylacetat extrahiert. Die organischen Phasen werden über wasserfreiem Natriumsulfat getrocknet. Nach Abdestillieren des Lösungsmittels wird das zurückbleibende Öl an Kieselgel unter Verwendung von Diethylether/n-Hexan (1:1) als Laufmittel chromtographisch gereinigt. Man erhält auf diese Weise den [E]-3-Methoxy-2-[α-{[(methylbenzyl)imino]oxy}-o-tolyl)-acrylsäure-methylester als gelbes Öl.

### Beispiel 2 (Herstellungsbeispiel außerhalb des stofflichen Umfangs der Erfindung)

3,67 g (13,2 mMol) 2-(α-Brom-o-tolyl)-3-methoxyacrylsäure-methylester und 3,0 g (13,2 mMol) 4-Phenoxyacetophenonoxim in 13 ml Methylenchlorid werden mit 13 ml 2,2 N Natriumhydroxidlösung und 5,7 g gleichen Tetrabutylammoniumhydrogensulfat in 10 Minuten bei Raumtemperatur intensiv gerührt. Dann gibt man die gleichen Mengen Methylenchlorid, 2,2 N Natriumhydroxidlösung und Tetrabutylammoniumhydrogensulfat hinzu und rührt weitere 10 Minuten. Anschließend werden nochmals die gleichen Mengen 2,2 N Natriumhydroxidlösung und Tetrabutylammoniumhydrogensulfat zugegeben und nach weiteren 10 Minuten Rühren gesättigte Natriumhydrogencarbonatlösung.

Man extrahiert das Gemisch dreimal mit Aethylacetat, wäscht die vereinigten organischen Phasen mit gesättigter Natriumchloridlösung und trocknet sie über wasserfreiem Natriumsulfat. Nach Abdestillieren des Lösungsmittels wird das zurückbleibende Öl an Kieselgel unter Verwendung von n-Hexan/Diethylether (1:1) als Laufmittel chromatographisch gereinigt:

Auf diese Weise erhält man den [E]-3-Methoxy-2-{α-{[(α-methyl-4-phenoxybenzyl)imino]oxy}-o-tolyl]-acrylsäure-methylester als gelbes Öl.

### Beispiel 3 - 123

Analog dem Beispiel 1 ("Methode 1") oder Beispiel 1 ("Methode 2") beschriebenen Verfahren erhält man ausgehend von 2-(α-Brom-o-tolyl)-3-methoxyacrylsäuremethylester und dem entsprechenden Oxim der Formel II die in der nachfolgenden Tabelle aufgeführten Verbindungen der Formel I':

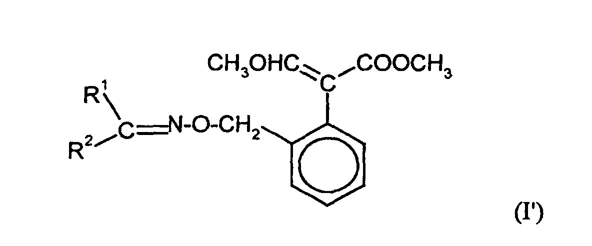

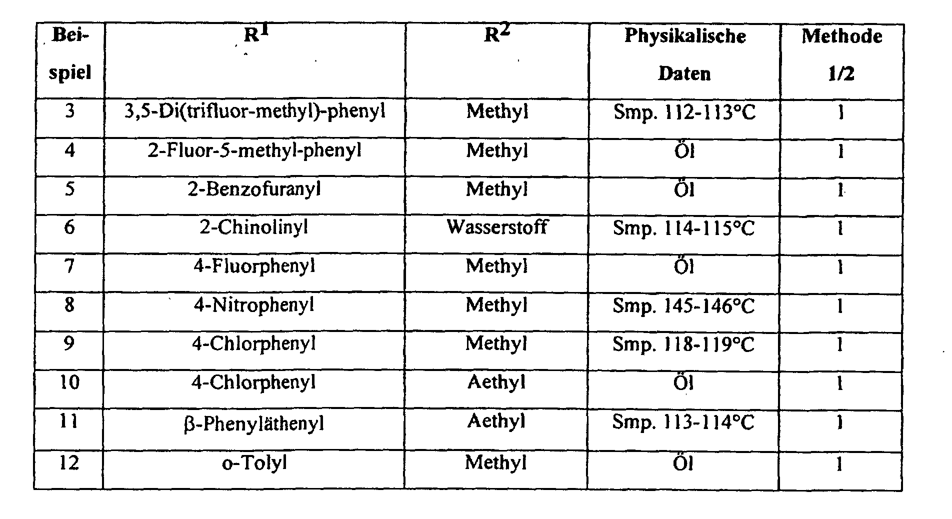

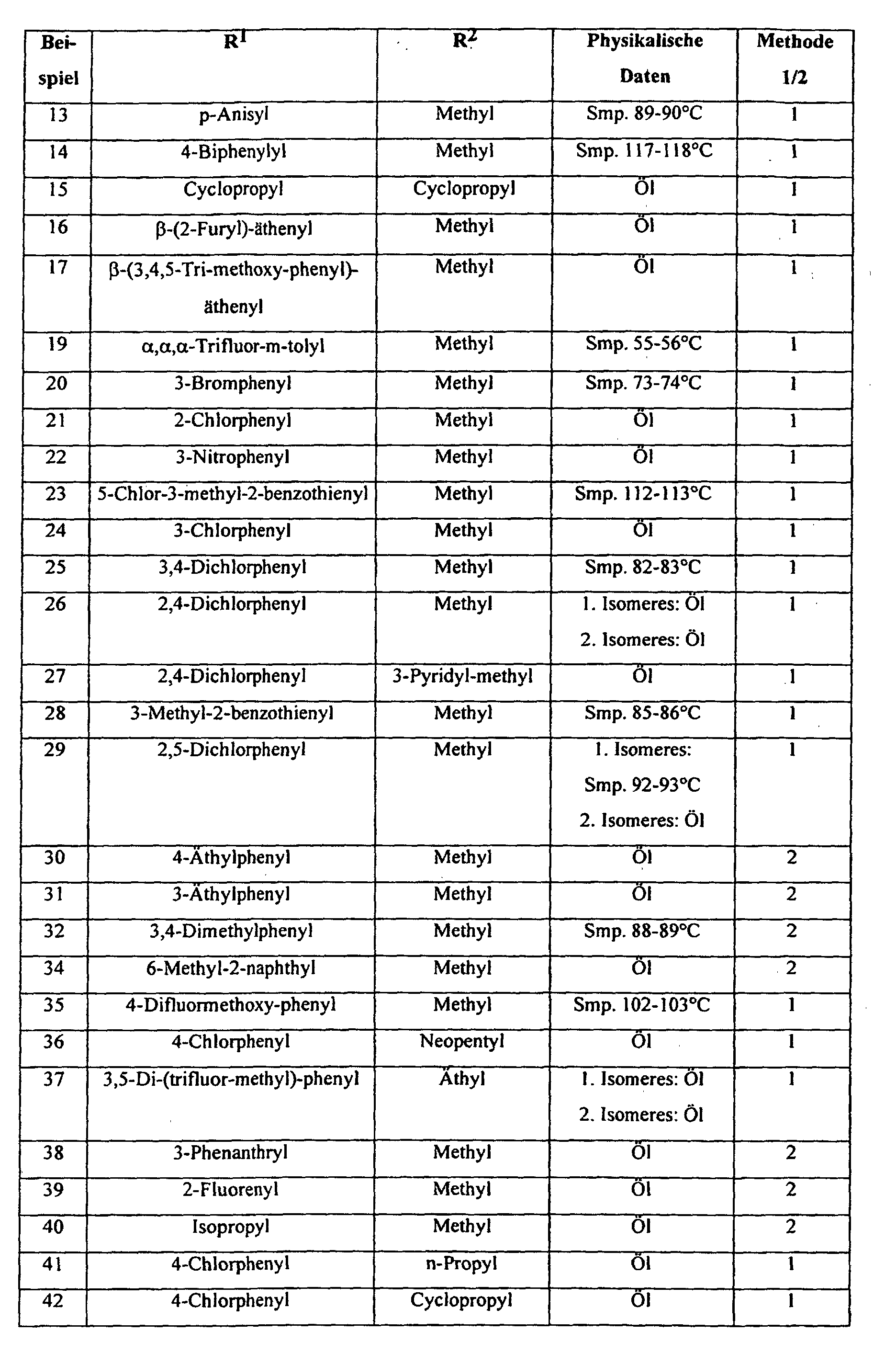

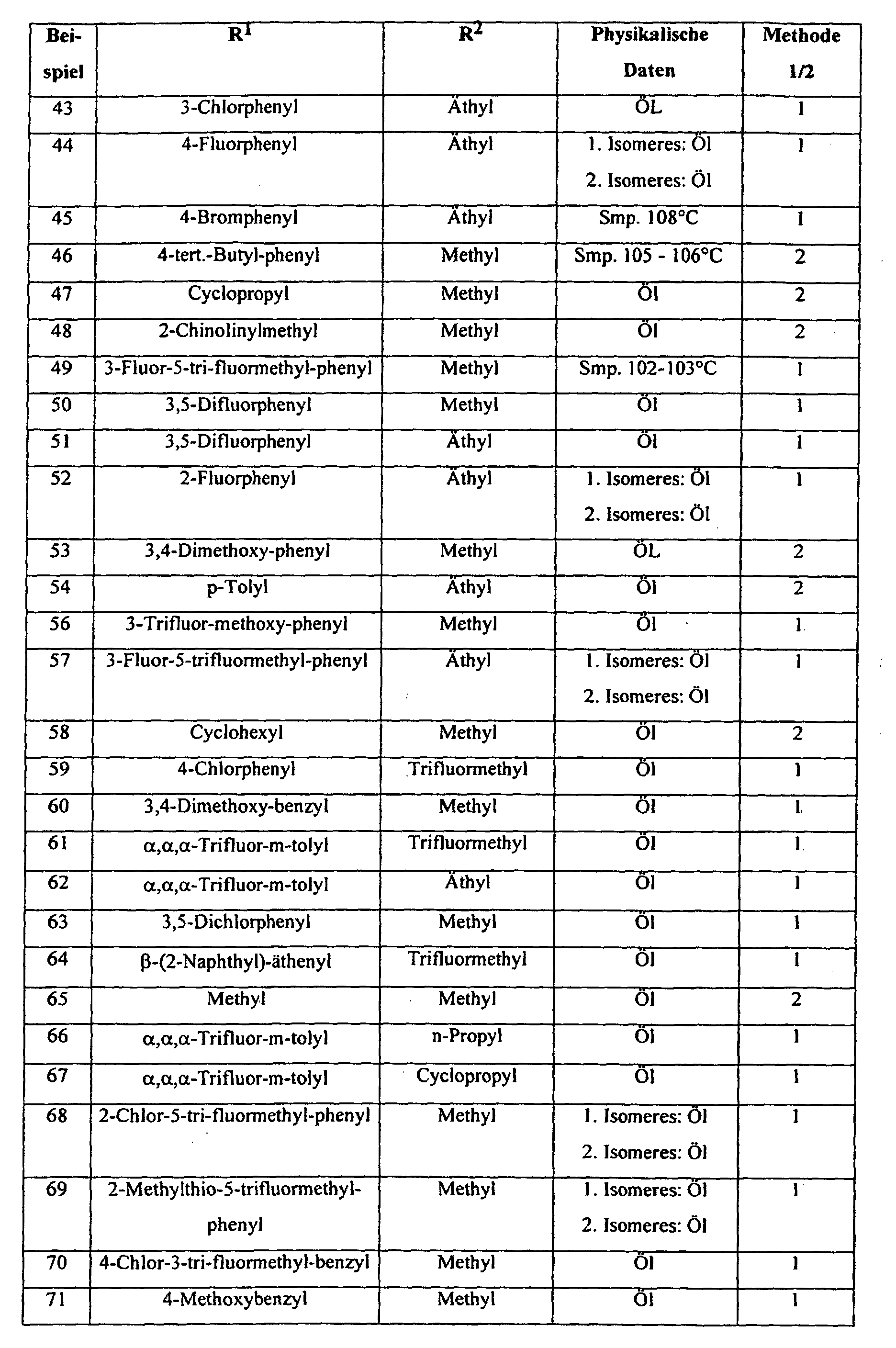

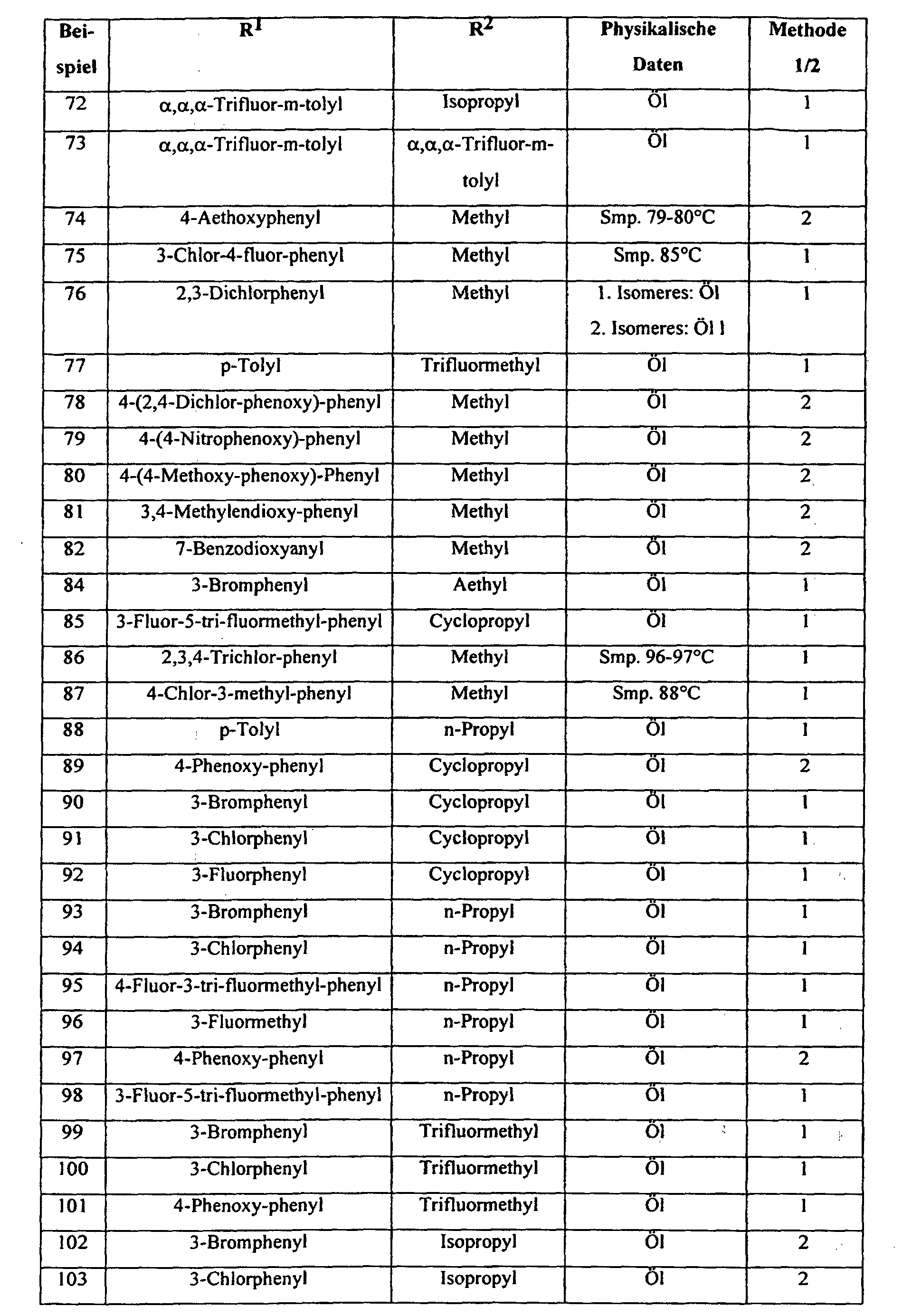

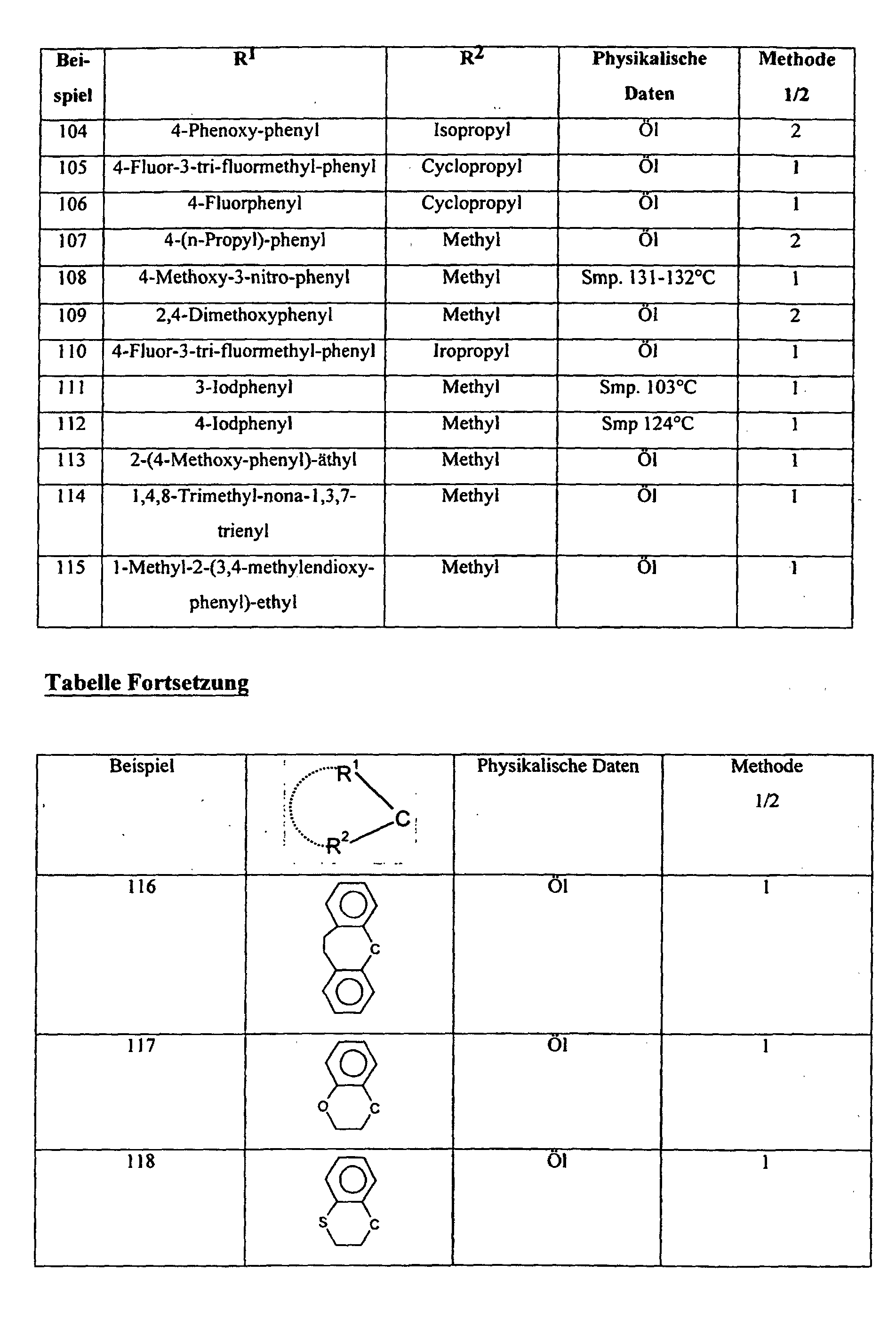

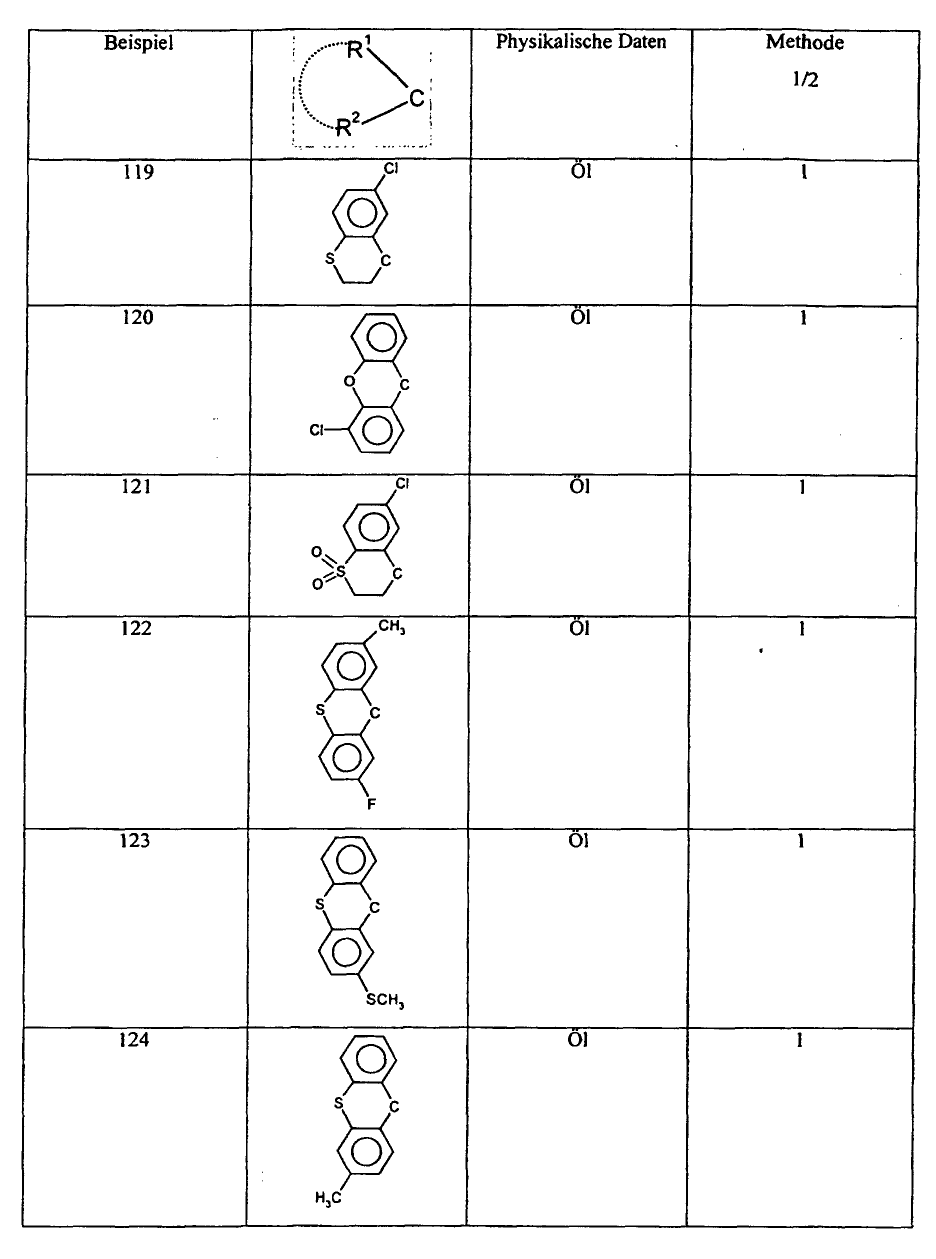

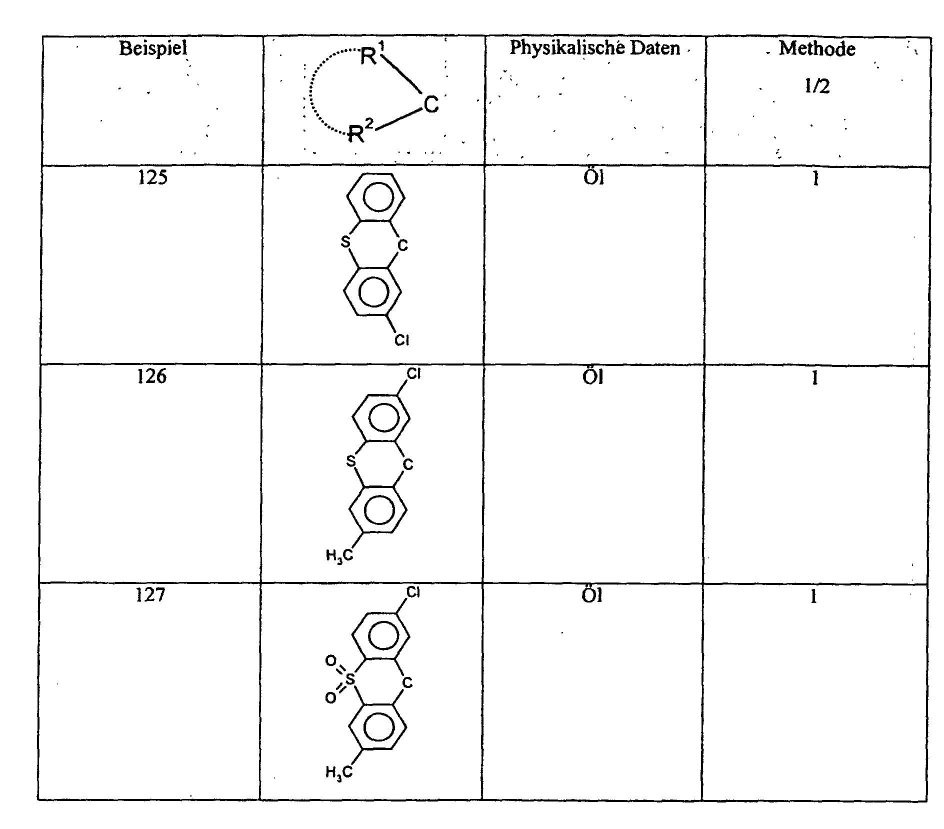

### II. Formulierungsbeispiele:

### Beispiel 245

Ein emulgierbares Konzentrat hat folgende Zusammensetzung:

| | g/Liter |
|---|---|
| Wirkstoff (erfindungsgemäße Verbindung) | 100 |
| Nonylphenol-(10)äthoxylat (nicht ionischer Emulgator) | 50 |
| Calcium-dodecylbenzolsulfonat (anionischer Emulgator) | 25 |
| N-Methyl-2-pyrrolidon (Lösungsvermittler) | 200 |
| Gemisch von Alkylbenzolen (Lösungsmittel) | ad 1 l |

Der Wirkstoff und die Emulgatoren werden im Lösungsmittel und im Lösungsvermittler gelöst. Durch Emulgieren dieses Konzentrates in Wasser kann eine gebrauchsfertige Spritzbrühe beliebiger Konzentration hergestellt werden.

### Beispiel 246

Ein Spitzpulver hat folgende Zusammensetzung:

| | Gew.-% |
|---|---|
| Wirkstoff (erfindungsgemäße Verbindung) | 25,0 |
| Kieselsäure (hydratisiert: Trägerstoff) | 20,0 |
| Natrium-laurylsulfat (Netzmittel) | 2,0 |
| Natrium-lignosulfonat (Dispergiermittel) | 4,0 |
| Kaolin (Trägerstoff) | 49,0 |

Die Komponenten werden miteinander vermischt, und das ganze wird in einer geeigneten Mühle feingemahlen. Durch Dispergieren des Gemisches in Wasser ergibt sich eine Suspension, die sich als gebrauchsfertige Spritzbrühe eignet.

## Patentansprüche

1. Verbindungen der allgemeinen Formel 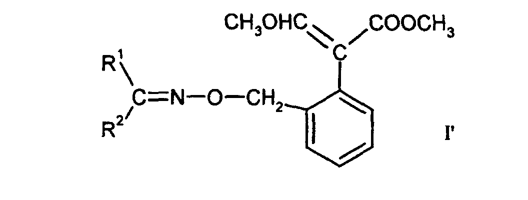 in welcher R¹ und R² die folgenden Bedeutungen haben:
| **R**^{**1**} | **R**^{**2**} |
|---|---|
| 3,5-Di(trifluormethyl)-phenyl | Methyl |
| 2-Fluor-5-methylphenyl | Methyl |
| 2-Benzofuranyl | Methyl |
| 2-Chinolinyl | Wasserstoff |
| 4-Fluorphenyl | Methyl |
| 4-Nitrophenyl | Methyl |
| 4-Chlorphenyl | Methyl |
| 4-Chlorphenyl | Aethyl |
| β-Phenyläthenyl | Methyl |
| o-Tolyl | Methyl |
| p-Anisyl | Methyl |
| 4-Biphenylyl | Methyl |
| Cyclopropyl | Cyclopropyl |
| β-(2-Furyl)-äthenyl | Methyl |
| β-(3,4,5-Trimethoxy-phenyl)-äthenyl | Methyl |
| α, α, α,-Trifluor-m-tolyl | Methyl |
| 3-Bromphenyl | Methyl |
| 2-Chlorphenyl | Methyl |
| 3-Nitrophenyl | Methyl |
| 5-Chlor-3-methyl-2-benzothienyl | Methyl |
| 3-Chlorphenyl | Methyl |
| 3,4-Dichlorphenyl | Methyl |
| 2,4-Dichlorphenyl | Methyl |
| 2,4-Dichlorphenyl | 3-Pyridylmethyl |
| 3-Methyl-2-benzothienyl | Methyl |
| 2,5-Dichlorphenyl | Methyl |
| 4-Aethylphenyl | Methyl |
| 3-Aethylphenyl | Methyl |
| 3,4-Dimethylphenyl | Methyl |
| 6-Methyl-2-naphthyl | Methyl |
| 4-Difluormethoxyphenyl | Methyl |
| 4-Chlorphenyl | Neopentyl |
| 3,5-Di(trifluormethyl)-phenyl | Aethyl |
| 3-Phenanthryl | Methyl |
| 2-Fluorenyl | Methyl |
| Isopropyl | Methyl |
| 4-Chlorphenyl | n-Propyl |
| 4-Chlorphenyl | Cyclopropyl |
| 3-Chlorphenyl | Aethyl |
| 4-Fluorphenyl | Aethyl |
| 4-Bromphenyl | Aethyl |
| 4-tert.-Butylphenyl | Methyl |
| Cyclopropyl | Methyl |
| 2-Chinolinylmethyl | Methyl |
| 3-Fluor-5-trifluormethyl-phenyl | Methyl |
| 3,5-Difluorphenyl | Methyl |
| 3,5-Difluorphenyl | Aethyl |
| 2-Fluorphenyl | Aethyl |
| 3,4-Dimethoxyphenyl | Methyl |
| p-Tolyl | Aethyl |
| 3-Trifluormethoxyphenyl | Methyl |
| 3-Fluor-5-trifluormethyl-phenyl | Aethyl |
| Cyclohexyl | Methyl |
| 4-Chlorphenyl | Trifluormethyl |
| 3,4-Dimethoxybenzyl | Methyl |
| α, α, α,-Trifluor-m-tolyl | Trifluormethyl |
| α, α, α,-Trifluor-m-tolyl | Aethyl |
| 3,5-Dichlorphenyl | Methyl |
| β-(2-Naphthyl)-äthenyl | Trifluormethyl |
| Methyl | Methyl |
| α, α, α,-Trifluor-m-tolyl | n-Propyl |
| α, α, α,-Trifluor-m-tolyl | Cyclopropyl |
| 2-Chlor-5-trifluormethyl-phenyl | Methyl |
| 2-Methylthio-5-trifluormethylphenyl | Methyl |
| 4-Chlor-3-trifluormethyl-benzyl | Methyl |
| 4-Methoxybenzyl | Methyl |
| α, α, α,-Trifluor-m-tolyl | Isopropyl |
| α, α, α,-Trifluor-m-tolyl | α, α, α,-Trifluor-m-tolyl |
| 4-Äthoxyphenyl | Methyl |
| 3-Chlor-4-fluorphenyl | Methyl |
| 2,3-Dichlorphenyl | Methyl |
| p-Tolyl | Trifluormethyl |
| 4-(2,4-Dichlorphenoxy)-phenyl | Methyl |
| 4-(4-Nitrophenoxy)phenyl | Methyl |
| 4-(4-Methoxyphenoxy)-phenyl | Methyl |
| 3,4-Methylendioxyphenyl | Methyl |
| 7-Benzodioxanyl | Methyl |
| 3-Bromphenyl | Aethyl |
| 3-Fluor-5-trifluormethylphenyl | Cyclopropyl |
| 2,3,4-Trichlorphenyl | Methyl |
| 4-Chlor-3-methylphenyl | Methyl |
| p-Tolyl | n-Propyl |
| 4-Phenoxyphenyl | Cyclopropyl |
| 3-Bromphenyl | Cyclopropyl |
| 3-Chlorphenyl | Cyclopropyl |
| 3-Fluorphenyl | Cyclopropyl |
| 3-Bromphenyl | n-Propyl |
| 3-Chlorphenyl | n-Propyl |
| 4-Fluor-3-trifluormethylphenyl | n-Propyl |
| 3-Fluorphenyl | n-Propyl |
| 4-Phenoxyphenyl | n-Propyl |
| 3-Fluor-5-trifluormethylphenyl | n-Propyl |
| 3-Bromphenyl | Trifluormethyl |
| 3-Chlorphenyl | Trifluormethyl |
| 4-Phenoxyphenyl | Trifluormethyl |
| 3-Bromphenyl | Isopropyl |
| 3-Chlorphenyl | Isopropyl |
| 4-Phenoxyphenyl | Isopropyl |
| 4-Fluor-3-trifluormethylphenyl | Cyclopropyl |
| 4-Fluorphenyl | Cyclopropyl |
| 4-(n-Propyl)-phenyl | Methyl |
| 4-Methoxy-3-nitrophenyl | Methyl |
| 2,4-Dimethoxyphenyl | Methyl |
| 4-Fluor-3-trifluormethylphenyl | Isopropyl |
| 3-Iodphenyl | Methyl |
| 4-Iodphenyl | Methyl |
| 2-(4-Methoxyphenyl)äthyl | Methyl |
| 1,4,8-Trimethyl-nona-1,3,7-trienyl | Methyl |
| 1-Methyl-2-(3,4-methylendioxyphenyl)-äthyl | Methyl |

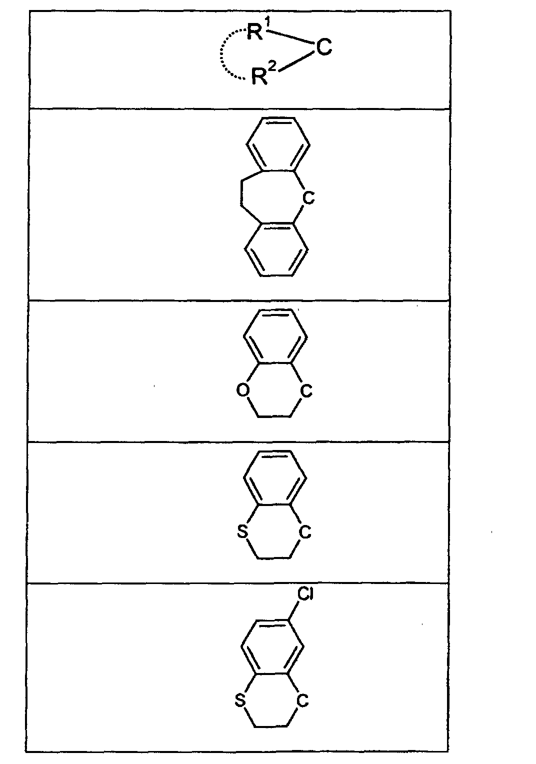

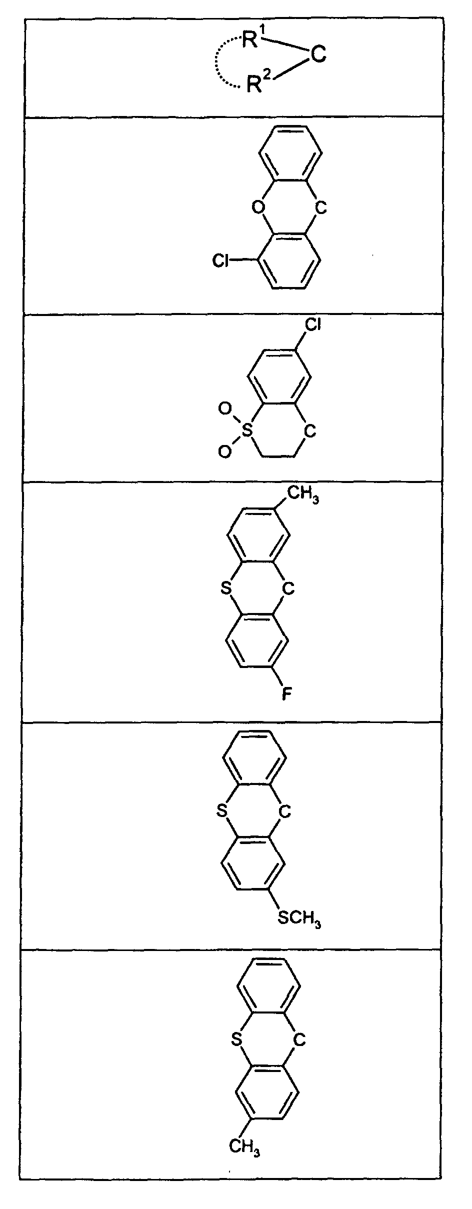

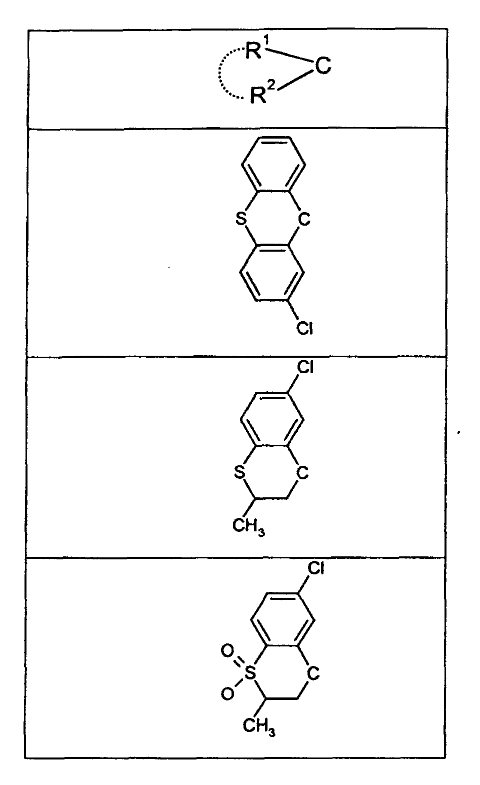

2. Fungizides Mittel, **dadurch gekennzeichnet, dass** es eine wirksame Menge mindestens einer Verbindung gemäß Anspruch 1 sowie Formulierungshilfsstoffe enthält.

3. Verfahren zur Herstellung einer Verbindung nach Anspruch 1 der allgemeinen Formel I' 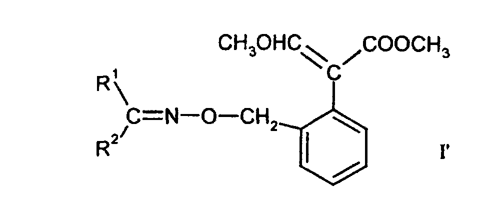 worin
R¹ und R² die in Anspruch 1 angegebenen Bedeutungen haben,
**dadurch gekennzeichnet, dass** man eine Verbindung der allgemeinen Formel 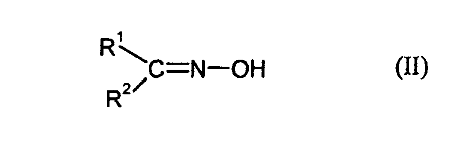 worin
R¹ und R² die in Anspruch 1 angegebenen Bedeutungen haben,
mit einem Benzylalkoholderivat der allgemeinen Formel 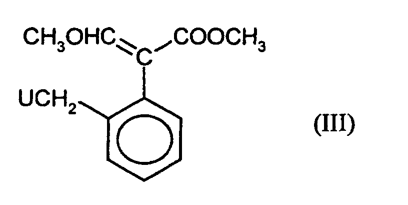 worin
U eine Abgangsgruppe bedeutet,
umsetzt.

4. Verfahren zur Bekämpfung von Fungi in der Landwirtschaft und im Gartenbau, **dadurch gekennzeichnet, dass** man das zu schützende Gut mit einer wirksamen Menge einer Verbindung gemäß Anspruch 1 bzw. eines Mittels gemäß Anspruch 1 behandelt.

5. Verwendung einer Verbindung gemäß Anspruch 1 bzw. eines Mittels gemäß Anspruch 2 zur Bekämpfung von Fungi in der Landwirtschaft und im Gartenbau.

## Claims

1. A compound of the general formula 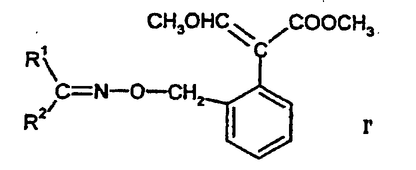 in which R¹ and R² have the following meanings:
| R¹ | R² |
|---|---|
| 3,5-di(trifluoromethyl)phenyl | methyl |
| 2-fluoro-5-methylphenyl | methyl |
| 2-benzofuranyl | methyl |
| 2-quinolinyl | hydrogen |
| 4-fluorophenyl | methyl |
| 4-nitrophenyl | methyl |
| 4-chlorophenyl | methyl |
| 4-chlorophenyl | ethyl |
| β-phenylethenyl | methyl |
| o-tolyl | methyl |
| p-anisyl | methyl |
| 4-biphenylyl | methyl |
| cyclopropyl | cyclopropyl |
| β-(2-furyl)ethenyl | methyl |
| β-(3,4,5-trimethoxyphenyl)ethenyl | methyl |
| α,α,α-trifluoro-m-tolyl | methyl |
| 3-bromophenyl | methyl |
| 2-chlorophenyl | methyl |
| 3-nitrophenyl | methyl |
| 5-chloro-3-methyl-2-benzothienyl | methyl |
| 3-chlorophenyl | methyl |
| 3-4-dichlorophenyl | methyl |
| 2,4-dichlorophenyl | methyl |
| 2,4-dichlorophenyl | 3-pyridylmethyl |
| 3-methyl-2-benzothienyl | methyl |
| 2,5-dichlorophenyl | methyl |
| 4-ethylphenyl | methyl |
| 3-ethylphenyl | methyl |
| 3,4-dimethylphenyl | methyl |
| 6-methyl-2-naphthyl | methyl |
| 4-difluoromethoxyphenyl | methyl |
| 4-chlorophenyl | neopentyl |
| 3,5-di(trifluoromethyl)phenyl | ethyl |
| 3-phenanthryl | methyl |
| 2-fluorenyl | methyl |
| isopropyl | methyl |
| 4-chlorophenyl | n-propyl |
| 4-chlorophenyl | cyclopropyl |
| 3-chlorophenyl | ethyl |
| 4-fluorophenyl | ethyl |
| 4-bromophenyl | ethyl |
| 4-tert-butylphenyl | methyl |
| cyclopropyl | methyl |
| 2-quinolinylmethyl | methyl |
| 3-fluoro-5-trifluoromethylphenyl | methyl |
| 3,5-difluorophenyl | methyl |
| 3,5-difluorophenyl | ethyl |
| 2-fluorophenyl | ethyl |
| 3,4-dimethoxyphenyl | methyl |
| p-tolyl | ethyl |
| 3-trifluoromethoxyphenyl | methyl |
| 3-fluoro-5-trifluoromethylphenyl | ethyl |
| cyclohexyl | methyl |
| 4-chlorophenyl | trifluoromethyl |
| 3,4-dimethoxybenzyl | methyl |
| α,α,α-trifluoro-m-tolyl | trifluoromethyl |
| α,α,α-trifluoro-m-tolyl | ethyl |
| 3,5-dichlorophenyl | methyl |
| β-(2-naphthyl)ethenyl | trifluoromethyl |
| methyl | methyl |
| α,α,α-trifluoro-m-tolyl | n-propyl |
| α,α,α-trifluoro-m-tolyl | cyclopropyl |
| 2-chloro-5-trifluoromethylphenyl | methyl |
| 2-methylthio-5-trifluoromethylphenyl | methyl |
| 4-chloro-3-trifluoromethylbenzyl | methyl |
| 4-methoxybenzyl | methyl |
| α,α,α-trifluoro-m-tolyl | isopropyl |
| a,a,a-trifluoro-m-tolyl | α,α,α,trifluoro-m-tolyl |
| 4-ethoxyphenyl | methyl |
| 3-chloro-4-fluorophenyl | methyl |
| 2,3-dichlorophenyl | methyl |
| p-tolyl | trifluoromethyl |
| 4-(2,4-dichlorophenoxy)phenyl | methyl |
| 4-(4-nitrophenoxy)phenyl | methyl |
| 4-(4-methoxyphenoxy)phenyl | methyl |
| 3,4-methylenedioxyphenyl | methyl |
| 7-benzodioxanyl | methyl |
| 3-bromophenyl | ethyl |
| 3-fluoro-5-trifluoromethylphenyl | cyclopropyl |
| 2,3,4-trichlorophenyl | methyl |
| 4-chloro-3-methylphenyl | methyl |
| p-tolyl | n-propyl |
| 4-phenoxyphenyl | cyclopropyl |
| 3-bromophenyl | cyclopropyl |
| 3-chlorophenyl | cyclopropyl |
| 3-fluorophenyl | cyclopropyl |
| 3-bromophenyl | n-propyl |
| 3-chlorophenyl | n-propyl |
| 4-fluoro-3-trifluoromethylphenyl | n-propyl |
| 3-fluorophenyl | n-propyl |
| 4-phenoxyphenyl | n-propyl |
| 3-fluoro-5-trifluoromethylphenyl | n-propyl |
| 3-bromophenyl | trifluoromethyl |
| 3-chlorophenyl | trifluoromethyl |
| 4-phenoxyphenyl | trifluoromethyl |
| 3-bromophenyl | isopropyl |
| 3-chlorophenyl | isopropyl |
| 4-phenoxyphenyl | isopropyl |
| 4-fluoro-3-trifluoromethylphenyl | cyclopropyl |
| 4-fluorophenyl | cyclopropyl |
| 4-(n-propyl)phenyl | methyl |
| 4-methoxy-3-nitrophenyl | methyl |
| 2,4-dimethoxyphenyl | methyl |
| 4-fluoro-3-trifluoromethylphenyl | isopropyl |
| 3-iodophenyl | methyl |
| 4-iodophenyl | methyl |
| 2-(4-methoxyphenyl)ethyl | methyl |
| 1,4,8-trimethyl-nona-1,3,7-trienyl | methyl |
| 1-methyl-2-(3,4-methylenedioxyphenyl)ethyl | methyl |

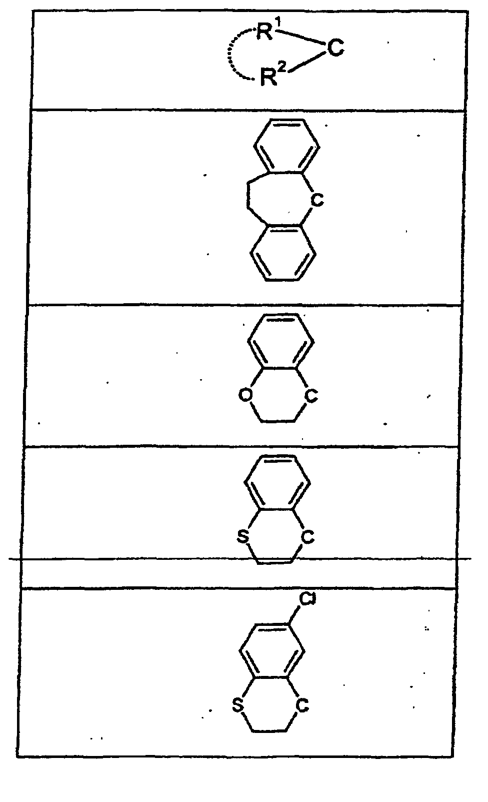

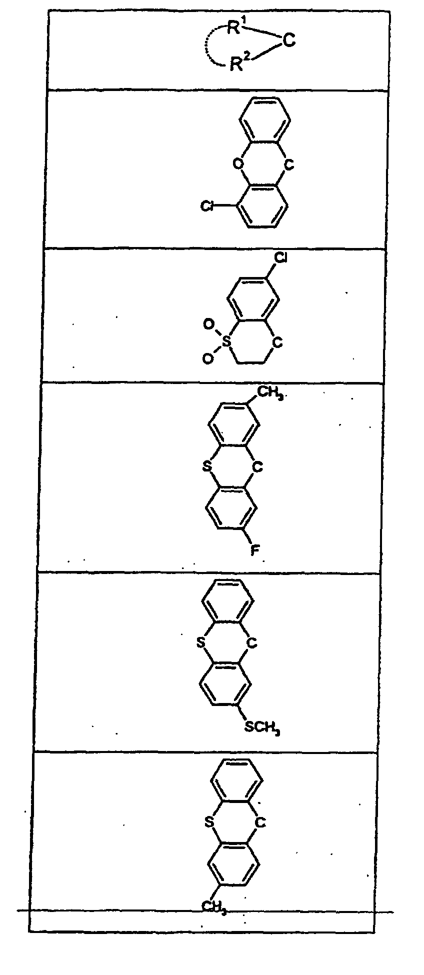

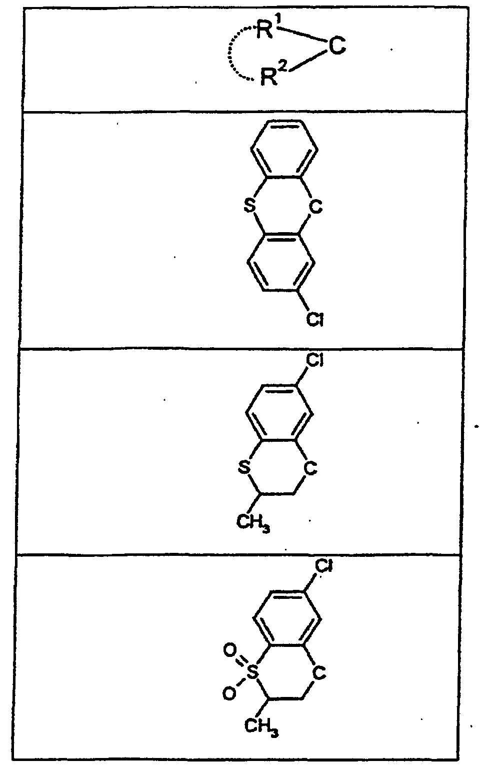

2. A fungicidal composition, **characterized in that** it contains an effective amount of at least one compound according to claim 1 as well as formulation adjuvants.

3. Process for the preparation of a compound as claimed in claim 1 of the general formula I' 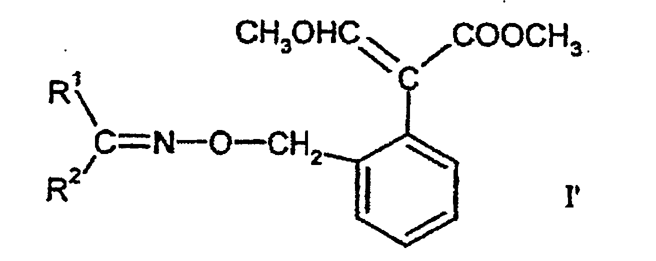 in which
R¹ and R² have the meanings given in Claim 1,
**characterized in that** a compound of the general formula 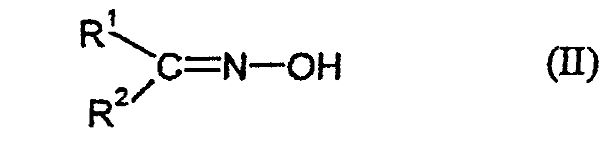 in which
R¹ and R² have the meanings given in Claim 1
are reacted with a benzyl alcohol derivative of the general formula 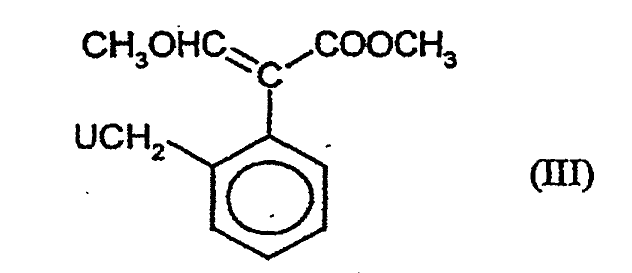 in which
U represents a leaving group.

4. A method for controlling fungi in agriculture and horticulture, **characterized in that** the locus to be protected is treated with an effective amount of a compound according to claim 1, or of a composition according to Claim 2.

5. The use of a compound according to Claim 1, or of a composition according to Claim 2, for controlling fungi in agriculture and horticulture.

## Revendications

1. Composés de formule générale 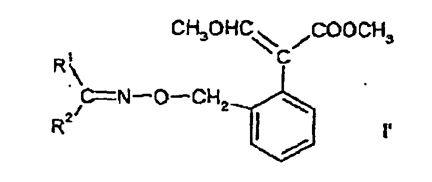 dans laquelle R¹ et R² ont la signification suivante :
| R¹ | R² |
|---|---|
| 3,5-di-(trifluorométhyl)-phényle | Méthyle |
| 2-fluoro-5-méthylphényle | Méthyle |
| 2-benzofurannyle | Méthyle |
| 2-quinoléinyle | Hydrogène |
| 4-fluorophényle | Méthyle |
| 4-nitrophényle | Méthyle |
| 4-chlorophényle | Méthyle |
| 4-chlorophényle | Ethyle |
| β-phényléthényle | Méthyle |
| o-tolyle | Méthyle |
| p-anisyle | Méthyle |
| 4-biphénylyle | Méthyle |
| Cyclopropyle | Cyclopropyle |
| β-(2-furyl)-éthényle | Méthyle |
| β-(3,4,5-triméthoxyphényl)-éthényle | Méthyle |
| α, α, α-trifluoro-m-tolyle | Méthyle |
| 3-bromophényle | Méthyle |
| 2-chlorophényle | Méthyle |
| 3-nitrophényle | Méthyle |
| 5-chloro-3-méthyl-2-benzothiényle | Méthyle |
| 3-chlorophényle | Méthyle |
| 3,4-dichlorophényle | Méthyle |
| 2,4-dichlorophényle | Méthyle |
| 2,4-dichlorophényle | 3-pyridylméthyle |
| 3-méthyl-2-benzothiényle | Méthyle |
| 2,5-dichlorophényle | Méthyle |
| 4-éthylphényle | Méthyle |
| 3-éthylphényle | Méthyle |
| 3,4-diméthylphényle | Méthyle |
| 6-méthyl-2-naphtyle | Méthyle |
| 4-difluorométhoxyphényle | Méthyle |
| 4-chlorophényle | Néopentyle |
| 3,5-di-(trifluorométhyl)-phényle | Ethyle |
| 3-phénantryle | Méthyle |
| 2-fluorényle | Méthyle |
| Isopropyle | Méthyle |
| 4-chlorophényle | n-propyle |
| 4-chlorophényle | Cyclopropyle |
| 3-chlorophényle | Ethyle |
| 4-fluorophényle | Ethyle |
| 4-bromophényle | Ethyle |
| 4-tert-butylphényle | Méthyle |
| Cyclopropyle | Méthyle |
| 2-quinoléinylméthyle | Méthyle |
| 3-fluoro-5-trifluorométhylphényle | Méthyle |
| 3,5-difluorophényle | Méthyle |
| 3,5-difluorophényle | Ethyle |
| 2-fluoroohényle | Ethyle |
| 3,4-diméthoxyphényle | Méthyle |
| p-tolyle | Ethyle |
| 3-trifluorométhoxyphényle | Méthyle |
| 3-fluoro-5-trifluorométhylphényle | Ethyle |
| Cyclohexyle | Méthyle |
| 4-chlorophényle | Trifluorométhyle |
| 3,4-diméthoxybenzyle | Méthyle |
| α, α, α-trifluoro-m-tolyle | Trifluorométhyle |
| α, α, α-trifluoro-m-tolyle | Ethyle |
| 3,5-dichlorophényle | Méthyle |
| β-(2-naphtyl)éthényle | Trifluorométhyle |
| Méthyle | Méthyle |
| α, α, α-trifluoro-m-tolyle | n-propyle |
| α, α, α-trifluoro-m-tolyle | Cyclopropyle |
| 2-chloro-5-trifluorométhylphényle | Méthyle |
| 2-méthylthio-5-trifluorométhylphényle | Méthyle |
| 4-chloro-3-trifluorométhylbenzyle | Méthyle |
| 4-méthoxybenzyle | Méthyle |
| α, α, α-trifluoro-m-tolyle | Isopropyle |
| α, α, α-trifluoro-m-tolyle | α, α, α-trifluoro-m-tolyle |
| 4-éthoxyphényle | Méthyle |
| 3-chloro-4-fluorophényle | Méthyle |
| 2,3-dichlorophényle | Méthyle |
| p-tolyle | Trifluorométhyle |
| 4-(2,4-dichlorophénoxy)-phényle | Méthyle |
| 4-(4-nitrophénoxy)-phényle | Méthyle |
| 4-(4-méthoxyphénoxy)-phényle | Méthyle |
| 3,4-méthylènedioxyphényle | Méthyle |
| 7-benzodioxannyle | Méthyle |
| 3-bromophényle | Ethyle |
| 3-fluoro-5-trifluorométhylphényle | Cyclopropyle |
| 2,3,4-trichlorophényle | Méthyle |
| 4-chloro-3-méthylphényle | Méthyle |
| p-tolyle | n-propyle |
| 4-phénoxyphényle | Cyclopropyle |
| 3-bromophényle | Cyclopropyle |
| 3-chlorophényle | Cyclopropyle |
| 3-fluorophényle | Cyclopropyle |
| 3-bromophényle | n-propyle |
| 3-chlorophényle | n-propyle |
| 4-fluoro-3-trifluorométhylphényle | n-propyle |
| 3-fluorophényle | n-propyle |
| 4-phénoxyphényle | n-propyle |
| 3-fluoro-5-trifluorométhylphényle | n-propyle |
| 3-bromophényle | Trifluorométhyle |
| 3-chlorophényle | Trifluorométhyle |
| 4-phénoxyphényle | Trifluorométhyle |
| 3-bromophényle | Isopropyle |
| 3-chlorophényle | Isopropyle |
| 4-phénoxyphényle | Isopropyle |
| 4-fluoro-3-trifluorométhylphényle | Cyclopropyle |
| 4-fluorophényle | Cyclopropyle |
| 4-(n-propyl)-phényle | Méthyle |
| 4-méthoxy-3-nitrophényle | Méthyle |
| 2,4-diméthoxyphényle | Méthyle |
| 4-fluoro-3-trifluorométhylphényle | Isopropyle |
| 3-iodophényle | Méthyle |
| 4-iodophényle | Méthyle |
| 2-(4-méthoxyphényl)-éthyle | Méthyle |
| 1,4,8-triméthyl-nona-1,3,7-triényle | Méthyle |
| 1-méthyl-2-(3,4-méthylènedioxyphényl)-éthyle | Méthyle |

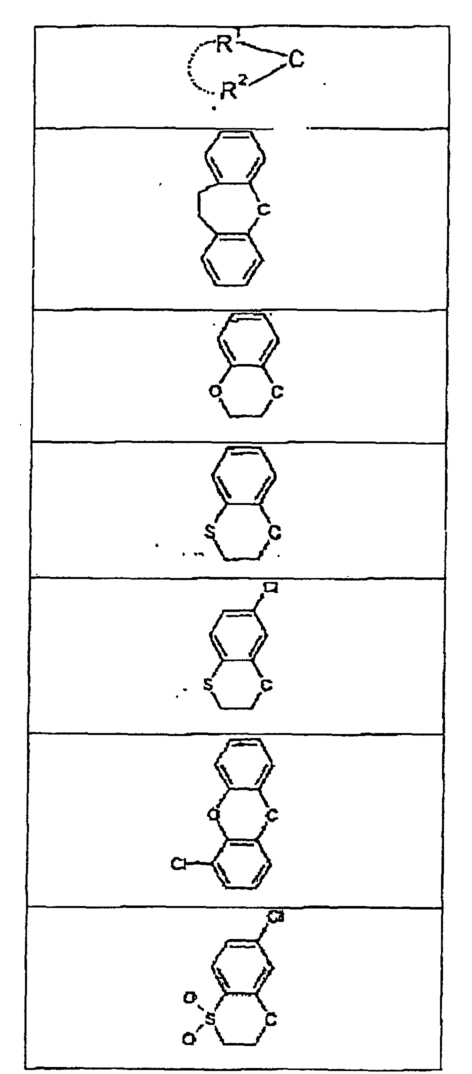

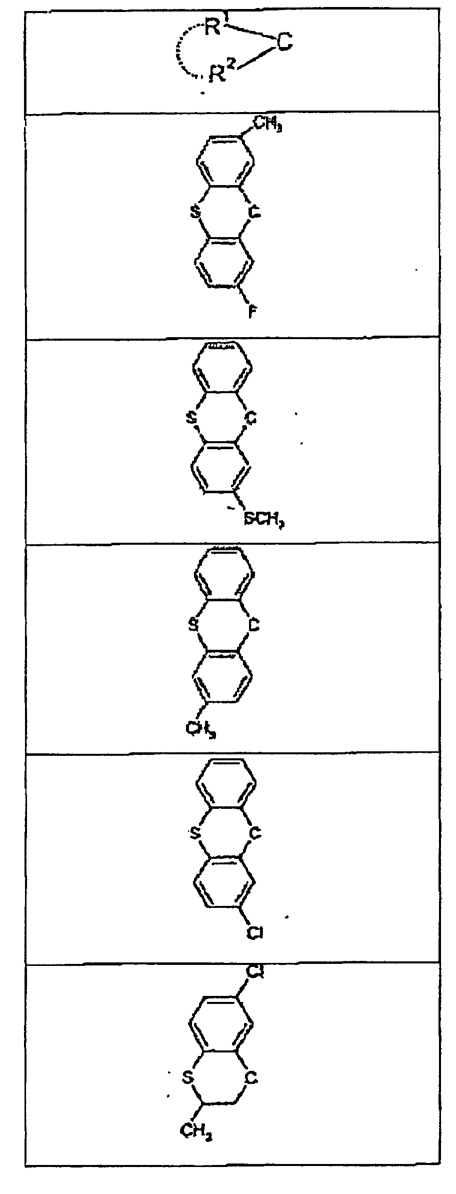

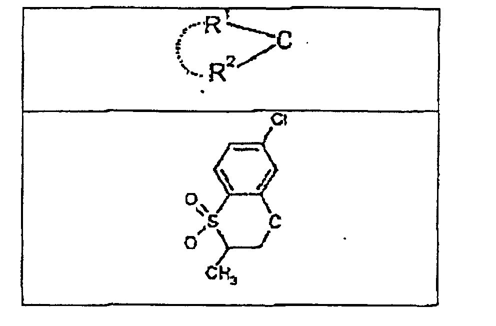

2. Produit fongicide, **caractérisé en ce qu'** il contient une quantité efficace d'au moins un composé selon la revendication 1 ainsi que des produits auxiliaires de formulation.

3. Procédé de préparation d'un composé de formule générale I' selon la revendication 1. 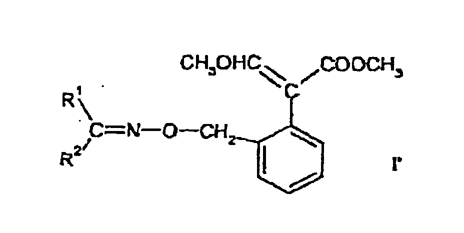 dans laquelle
R¹ et R² ont la signification donnée dans la revendication 1,
**caractérisé en ce que** l'on fait réagir un composé de formule générale 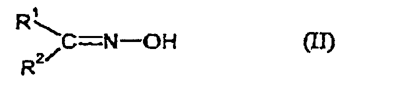 dans laquelle R¹ et R² ont la signification donnée dans la revendication 1,
avec un dérivé de l'alcool benzylique de formule générale 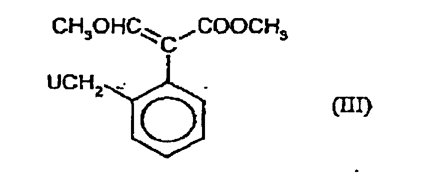 dans laquelle
U représente un groupe éliminable.

4. Procédé pour combattre les mycètes dans l'agriculture et l'horticulture, **caractérisé en ce que** l'on traite l'objet à protéger par une quantité efficace d'un composé selon la revendication 1 ou d'un produit selon la revendication 2.

5. Utilisation d'un composé selon la revendication 1 ou d'un produit selon la revendication 2 pour la lutte contre les mycètes dans l'agriculture et l'horticulture.
